Europäisches Patentamt

European Patent Office

Office européen des brevets

(11) Publication number : **0 489 497 A1**

## (12) EUROPEAN PATENT APPLICATION

(21) Application number : **91310185.3**

(51) Int. Cl.⁵ : **C07C 11/02, C07C 1/20**

(22) Date of filing : **04.11.91**

(30) Priority : **08.11.90 GB 9024343**

(43) Date of publication of application :
**10.06.92 Bulletin 92/24**

(84) Designated Contracting States :
**AT BE CH DE DK ES FR GB GR IT LI LU NL SE**

(71) Applicant : **THE BRITISH PETROLEUM COMPANY P.L.C.**
**Britannic House, 1 Finsbury Circus**
**London EC2M 7BA (GB)**

(72) Inventor : **Barri, Sami Ali Ibrahim**
**The British Petroleum Co.p.l.c., Chertsey Road**
**Sunbury-on-Thames, Middlesex TW16 7LN**
**(GB)**

(74) Representative : **Scott, Susan Margaret et al**
**BP INTERNATIONAL LIMITED Patents &**
**Agreements Division Chertsey Road**
**Sunbury-on-Thames Middlesex TW16 7LN**
**(GB)**

(54) **Process for the preparation of branched olefins.**

(57)   A process for the production of olefins which comprises passing an oxygenate- containing feedstock over a zeo type catalyst at a temperature greater than 200°C, characterised in that the feedstock comprising an olefin and, as oxygenate, methanol, formaldehyde and/or dimethylether, the zeo type catalyst is of TON-type structure, and the feedstock includes added water.

EP 0 489 497 A1

The present invention relates to a catalytic process for the production of branched olefins by utilising a zeolite catalyst in the conversion of oxygenates, especially methanol, in the presence of olefins.

It is known from EP-A-65400 that a zeolite of TON-type structure (referred to in that document as Nu-10) is able to convert methanol into olefins. However, experiments have shown that this reaction is inefficient as the catalyst deactivates after a short period of time.

US 4684757 also describes a methanol to olefins process. In this process, ethene and/or propene are amongst the products produced, and these are then recycled to the reaction. Although zeolite ZSM-5 is the preferred catalyst for the reaction disclosed in US 4684757, other zeolites are also listed: these zeolites are ZSM-11, ZSM-12, ZSM-22, ZSM-23, ZSM-35, ZSM-38, ZSM-45 and ZSM-50. ZSM-22 is of TON-type structure. In fact, ZSM-22 would not be a suitable catalyst for the process of US 4684757: as stated above, the catalyst deactivates after a short period of time when using an initial feed of methanol.

The conversion of methanol to olefins over ZSM-5 is very well known. In addition to US 4684757 described above, the following documents are of interest. US 454335 describes a process in which ethene is recycled in an amount of up to 20 parts by weight ethene to 100 parts by weight methanol. US 4579999 discloses a similar process wherein gasoline range olefins of $C_5$ and above are recycled to the reaction process. Such processes using ZSM-5 convert methanol into a wide spectrum of olefinic products plus gasoline, the precise product composition depending of course on the reaction conditions used. Thus, US 4543435 at column 4 lines 43 onwards gives yields obtained in a fluidised bed using ZSM-5 catalyst. The major products obtained are $C_5$ and higher gasoline-range hydrocarbons, with other products over a range of carbon numbers also being produced. Ethene recycle makes almost no difference to the product distribution.

We have now found a method for the very selective production of $C_4/C_5$ olefins. This method uses a feed containing methanol, formaldehyde and/or dimethyl ether, together with a $C_3$ and/or $C_4$ olefin, and a TON-type zeolite as a catalyst. Unlike prior art methods of using TON-type catalysts, catalyst deactivation is significantly retarded. The product spectrum obtained is completely different from that obtained using ZSM-5.

Accordingly, the present invention provides a process for the production of olefins which comprises passing an oxygenate-containing feedstock over a zeo type catalyst at a temperature greater than 200°C, characterised in that the feedstock comprising an olefin and, as oxygenate, methanol, formaldehyde and/or dimethylether, the zeo type catalyst is of TON-type structure, and the feedstock includes added water.

The process provides a product which is rich in branched olefinic hydrocarbons.

Throughout this specification and claims, "oxygenate" means methanol, formaldehyde and/or dimethylether.The feedstock comprises oxygenate and a proportion of an olefin. Especially preferred are $C_3$ and/or $C_4$ olefins. The oxygenate and olefin are preferably present in the feedstock in molar ratio of greater than 1 mole olefin to 20 moles oxygenate, preferably greater than 1 mole olefin to 10 moles oxygenate, especially greater than 1 mole olefin to 5 moles oxygenate, most preferably greater than 1 mole olefin to 4 moles oxygenate. Preferably the feedstock contains at least 1 mole oxygenate to 20 moles olefin. The ratio could be chosen suitably according to the conditions and catalyst composition employed to maximise the production of the desired branched olefins, especially isobutene and methylbutenes. The preferred oxygenate is methanol.

The presence of water in the feedstock has unexpected and beneficial effects especially reducing the level of undesired high molecular weight hydrocarbons in the product. It should be understood that the water referred to in the present invention is added over and above the quantities of water normally present in commercial grades of the reactants. It is also of course over and above the quantities of water actually formed in the reaction. The level of water co-feed may be suitably less than 90% of total molar content, preferably less than 80% of total molar content, more preferably 5-70% of total molar content.

The feedstock may enter the reaction chamber as a single pre-mix. Equally suitable, the components may be fed in separately and mixed thereafter in the reaction chamber. The feedstock may, if desired be diluted with, for example, alkanes or an inert gas.

A code consisting of three capital letters has been adopted for each known structure type following the recommendations by IUPAC on zeolite nomenclature ("Chemical Nomenclature and Formulation of Compositions of Synthetic and Natural Zeolites", IUPAC, yellow booklet, 1978). TON-type structures are disclosed in the Atlas of Zeolite Structure Types by Meier WM and Olsen DH, 1987 distributed by Polycrystal Book Service, Pittsburgh USA. Such TON-type structures have uni-dimensional, non-intersecting channels with ten-membered ring openings of about 6Å in diameter.

The reaction may be carried out over zeo-type catalysts that have the TON-type structure eg aluminosilicates, gallosilicates, zincosilicates, borosilicates, titanosilicates etc or their germanate counterparts. The preferred zeo-type catalyst is an aluminosilicate.

For simplicity, the specification relates to the preparation and use of aluminosilicates. It should, of course be understood that the additional aforementioned TON-type structures may also be used and can be prepared in an analogous manner.

Zeolites having TON type structures are also known by the names Theta-1 which is disclosed in our European patent 57049, Nu-10 which is disclosed in the European patent 65400 and ZSM-22 which is disclosed in the Canadian Patent No 1202941.

The zeolite is suitably prepared from an initial mixture containing a source of silica, a source of alumina, a source of alkali metal(s), water and either an organic nitrogen containing base, as discussed in the European patents above or an inorganic nitrogen base as discussed in EP-A-104800.

The zeolite may be prepared by forming a mixture of all the reactants as described in the above documents. The mixture is then crystallised at a temperature above 70°C, preferably between 100 and 200°C for suitably at least 2 hours, preferably 6 to 240 hours. The optimum crystallisation period can vary and may depend upon such factors as the temperature, pH and gel composition. Preferably, the source of silica is an amorphous silica sol which is diluted with water. It is preferred that the silica source is added to the other reagents in such a way as to commence gelation at a relatively high PH.

The zeolite may vary in composition depending on the method of synthesis eg the Si/Al of the product may be varied by controlling the Si/Al ratio of the hydrogel precursor or by varying the OH/Si ratio.

The zeolite produced contains cations which, depending upon the precise synthesis method used, may be hydrogen, aluminium, alkali metals, organic nitrogen containing cations or any combination thereof.

The zeolite is preferably used in the present process in the hydrogen form. The hydrogen form may be achieved by, in the case of organic containing zeolite, calcination to remove the organics followed by either ammonium ion exchange followed by calcination, proton exchange with an acid solution or a combination of both. In the case of a zeolite synthesised in the absence of organic nitrogen containing compound the hydrogen form could, if desired, be prepared by either direct ammonium ion exchange followed by calcination or proton exchange with acid solution or a combination of both. The preparation of thehydrogen form of the zeolite may vary to maximise the production of isobutene and methylbutenes. If so desired, the hydrogen form of the zeolite also may be partially or completely exchanged or impregnated with a metal such as Ga or Mg and used in the present process.

The zeolite may be modified to alter its acidity or shape selectivity in such a way to improve the catalytic performance the modifications may include a calcination regime, steam treatment, chemical treatment eg with dealuminating agent eg $SiCl_4$, EDTA, etc aluminating agent eg sodium aluminate, $AlCl_3$ etc, inclusion of phosphorous compound, Lewis base, HF etc. A combination of treatments may also be carried out. The zeolite may be treated during the preparation of the H-form or be carried out on the H-form.

The zeolite may, if desired, be bound in a suitable binding material either before or after impregnation or after exchange with a metal compound. The binder may suitably be one of the conventional alumina, silica, clay, or aluminophosphate binders or a combination of binders.

The process according to the invention is carried out at a temperature in excess of 200°C, preferably 250 to 600 °C and may be carried out at reduced or elevated pressure relative to atmospheric pressure. Suitably, a pressure of from 0.1 - 100 bar absolute, preferably from 0.5 - 10 bar absolute and most preferably from 2-10 bar absolute may be used.

The feedstock is fed into the reaction chamber either with or without additional diluents eg alkanes or inert gas at a rate of suitably 0.1 - 1000 weight hourly space velocity (WHSV). Preferably, the WHSV is at least 2, more preferably at least 5, especially at least 10. WHSV's of up to 500, especially up to 100, are preferred. For the purposes of the present invention, it is understood that weight hourly space velocity is defined as the weight of olefin and oxygenate fed per weight of catalyst per hour. In addition, the mole % of any diluent gas present in the feed may be up to 90%, preferably up to 70%, most preferably up to 60%. If a diluent is present, it is preferably present in an amount of at least 5%.

It is well known that zeolites and similar molecular sieves tend to concentrate the reactants and thus promote bimolecular reactions. Zeolites such as those having structures of the MFI or MEL types tend to produce high levels of oligomers, naphthenes, aromatics and alkanes all of which are produced due to the promotion of bimolecular reactions. In this invention it has been found that the formation of heavy hydrocarbons can be reduced by a combination of using a molecular sieve having TON-type structure and optimising the WHSV or the contact time of the reaction. The optimum WHSV would be dependent on the other operating conditions and the catalyst composition and pretreatment. The relative concentration of the reactants can be optimised by adjusting the hydrocarbons to oxygenates ratio. In addition the contact time is optimised at constant WHSV by dilution with inert gas or less reactive gas than the reactants.

The process may be carried out in any suitable reactor, for example a fixed bed, fluid bed a slurry reactor or a continuous catalyst regeneration reactor.

The product of the process includes branched olefinic hydrocarbons; when the feedstock includes $C_3/C_4$ olefins, the product is rich in isobutene and methylbutene. A small amount of by-products eg methane, ethane and ethene are also present.

The products of the present process may be utilised as reactants in a second process, in particular, the etherification of branched olefins with an alcohol. The final products of the overall two-step process may suitably be methyl tertiarybutylether/tertiary amylmethylether mixtures and gasoline range hydrocarbons.

Aternatively, the products of the present invention may be further reacted to increase the degree of branching. Linear olefins produced may be isomerised to produce additional branched olefins. Oligomerisation of the small olefinic hydrocarbons may suitably produce highly branched longer olefins. Furthermore, alkylation of the linear olefins will suitably produce aliphatic hydrocarbons suitable for gasoline blending or if so desired, aromatisation of the linear olefins may suitably provide aromatic hydrocarbons suitable for gasoline blending.

The process will now be described with reference to the following examples.

Example 1 Synthesis of Theta-1 Zeolite

Theta-I was synthesised using ammonia as the templating agent. Sodium aluminate (30g, ex BDH, 40 wt% $Al_2O_3$, 30 wt% $Na_2O$ and 30 wt% $H_2O$) and sodium hydroxide (15.6g ex BDH) were dissolved in distilled water (240 g). Ammonia solution (1400g, SG 0.90° containing 25% $NH_3$) was added with gentle mixing. Ludox AS40 (Trade Mark) (1200g) which contained 40 wt% silica was added over fifteen minutes with stirring to maintain a homogeneous hydrogel. The molar composition of the hydrogel was:-

$$2.9 \ Na_2O:175 \ NH_3: 1.0 \ Al_2O_3:68 \ SiO_2:950 \ H_2O$$

The mixture was then loaded into a 5 litre Parr autoclave and crystallised at 175°C for 25 hours under autogeneous pressure whilst mixing by a mechanical stirring action. At the end of the crystallisation period the autoclave was cooled, and the product filtered, washed and dried in an air oven at 100°C. The crystallinity and the purity of the zeolite were determined by X-ray powder diffraction. The sample was Theta-1 with an estimated cristobalite content of less than 5%.

Example 2 Preparation of the H-form Theta-1 zeolite:

The Theta-1 as synthesised in Example 1 which contained both $Na^+$ and $NH_4^+$ ions was directly ion exchanged in order to remove the $Na^+$ ions. The zeolite was mixed for 1 hour with an aqueous ammonium nitrate solution (1M, zeolite to solution weight ratio of 1:20). The zeolite was filtered, washed and the ion exchange treatment repeated twice. The ammonium form of the zeolite was then dried at 100°C and calcined overnight in air at 550°C to convert to the hydrogen form. The X-ray diffraction pattern of the H-Form is shown in Table 1.

Example 3 Catalyst Preparation and Testing

The zeolite powder (H-form) was pressed into tablets at 10 tonnes. The tablets were broken and sieved into granules to pass 600 micron but not 250 micron sieves. 10cc of the catalyst granules (weight 4.2g) were loaded into a tubular reactor with a coaxial thermocouple well, activated in air at 550°C and tested for the conversion of various feedstocks. Table 2A provides the product stream analysis data obtained in the reaction between methanol and butene in the absence and presence of water. Table 2B provides the corresponding calculated conversion values. The results clearly show the benefits of added water, in the reduction of undesired high molecular weight hydrocarbons in the product.

The terms used in the Tables are defined as follows:

| | |
|---|---|
| Temperature | = applied temperature in °C |
| WHSV | = weight hourly space velocity which is the weight of the oxygenate and olefins fed per weight of the catalyst per hour |
| HOS | = hours on stream since the last air activations |
| Feed% | = molar feed compositions |
| MeOH | =Methanol |
| Conversion | = carbon molar conversions % of each feed |
| Selectivities | = carbon molar yield of each component x 100 total carbon molar conversions |
| $nC_4$ | = n-butene |
| $C_1/C_2$ | = methane, ethane and ethene |
| $C_3$ | = propane and propene |
| $iC_4$ | = iso-butene |
| $C_5$ | = pentenes |
| Cn | = hydrocarbons containing n carbon atoms per molecule |

4

$C_{6+}$ = hydrocarbons containing 6 or more carbon atoms per molecule

tr = trace (less than 0.005 detected)

### TABLE 1: XRD OF PRODUCT OF EXAMPLE 2

| 2 THETA (2θ) | D SPACINGS A° | RELATIVE INTENSITIES 100 x I/I°max |
|---|---|---|
| 8.17 | 10.81 | 100 |
| 10.16 | 8.70 | 22 |
| 12.81 | 6.91 | 23 |
| 16.36 | 5.42 | 11 |
| 19.42 | 4.57 | 12 |
| 20.36 | 4.36 | 97 |
| 24.22 | 3.67 | 82 |
| 24.64 | 3.61 | 52 |
| 25.76 | 3.46 | 36 |

Variation in intensities of ± 20%
Variation in 2θ peak positions of ± 0.2° with corresponding variation in D spacings.
Peaks below 10% of $I_{max}$ Excluded.
Copper alpha 1 wavelengths, 1.54060.

| | |
|---|---|
| X-Ray Diffractometer | Philips PW 1820/10 |
| Slits | 1/4°, 0.2°, 1/4° |
| 2θ Scan | 2° – 32° |
| Step Size | 0.025° |
| Time | 4 sec |

## TABLE 2A: PRODUCT STREAM ANALYSIS

| HOS | 1 | 4 | 6 | 1 | 4 | 6 | 4 |
|---|---|---|---|---|---|---|---|

### Feed composition (mmole/h)

| | 1 | 4 | 6 | 1 | 4 | 6 | 4 |
|---|---|---|---|---|---|---|---|
| 1-Butene | 16.61 | 16.98 | 16.31 | 16.09 | 16.12 | 15.98 | 627.8 |
| Methanol | 75.00 | 75.00 | 75.00 | 75.00 | 75.00 | 75.00 | 316.6 |
| Water | 0.0 | 0.0 | 0.0 | 166.67 | 166.67 | 166.67 | 1838.3 |

### Product composition (mmole/h)

| | 1 | 4 | 6 | 1 | 4 | 6 | 4 |
|---|---|---|---|---|---|---|---|
| Methane | 0.27 | 0.13 | 0.15 | 0.23 | 0.16 | 0.06 | 0.77 |
| Ethane | 0.01 | 0.01 | 0.01 | 0.0 | 0.0 | 0.0 | 0.06 |
| Ethene | 0.10 | 0.08 | 0.10 | 0.17 | 0.19 | 0.14 | 2.52 |
| Propane | 0.09 | 0.06 | 0.08 | 0.06 | 0.04 | 0.03 | 1.03 |
| Propene | 0.55 | 0.44 | 0.61 | 0.63 | 0.53 | 0.57 | 22.18 |
| i-Butane | 0.22 | 0.15 | 0.24 | 0.15 | 0.12 | 0.13 | 0.97 |
| n-Butane | 0.94 | 0.70 | 1.14 | 0.97 | 0.79 | 0.61 | 12.56 |
| t-2-Butene | 1.25 | 1.68 | 2.32 | 9.13 | 9.26 | 10.37 | 150.05 |
| 1-Butene | 0.58 | 0.32 | 0.89 | 3.88 | 3.70 | 4.30 | 104.09 |
| i-Butene | 1.39 | 1.04 | 1.73 | 1.76 | 1.47 | 1.56 | 87.22 |
| c-2-Butene | 0.88 | 0.85 | 1.37 | 6.04 | 6.33 | 6.84 | 112.70 |
| C5 | 1.89 | 1.78 | 2.36 | 0.51 | 0.64 | 0.47 | 19.15 |
| 2-Methylbutene-2 | 4.56 | 3.34 | 5.76 | 1.36 | 1.93 | 1.33 | 30.87 |
| 2-Methylbutene-1 | 1.27 | 1.60 | 1.77 | 0.48 | 0.61 | 0.44 | 13.41 |
| 3-Methylbutene-1 | 0.57 | 0.55 | 0.57 | 0.14 | 0.19 | 0.13 | 2.21 |
| C6 | 3.09 | 3.08 | 2.98 | 0.19 | 0.31 | 0.23 | 4.85 |
| C7 | 1.01 | 0.99 | 0.79 | 0.05 | 0.06 | 0.10 | 3.83 |
| C8 | 0.56 | 0.70 | 0.61 | 0.0 | 0.0 | 0.0 | 19.05 |
| C9 | 0.39 | 0.58 | 0.54 | 0.0 | 0.0 | 0.0 | 5.60 |
| C10 | 0.22 | 0.32 | 0.29 | 0.0 | 0.0 | 0.0 | 1.80 |
| C11 | 0.12 | 0.14 | 0.12 | 0.0 | 0.0 | 0.0 | 0.38 |
| C12 | 0.05 | 0.05 | 0.05 | 0.0 | 0.0 | 0.0 | 0.13 |
| C13 | 0.02 | 0.02 | 0.02 | 0.0 | 0.0 | 0.0 | 0.02 |
| C14 | 0.01 | 0.01 | 0.01 | 0.0 | 0.0 | 0.0 | 0.0 |
| C15 | tr | tr | tr | 0.0 | 0.0 | 0.0 | 0.0 |
| C16 | 0.0 | 0.0 | 0.0 | 0.0 | 0.0 | 0.0 | 0.0 |
| C17 | 0.0 | 0.0 | 0.0 | 0.0 | 0.0 | 0.0 | 0.0 |
| Carbon monoxide | 0.0 | 0.0 | 0.0 | 0.0 | 0.0 | 0.0 | 0.0 |
| Carbon dioxide | 0.0 | 0.0 | 0.0 | 0.0 | 0.0 | 0.0 | 0.0 |
| Methanol | 9.23 | 9.61 | 10.47 | 30.34 | 31.05 | 32.44 | 178.14 |
| Formaldehyde | 0.0 | 0.0 | 0.0 | 0.0 | 0.0 | 0.0 | 0.0 |
| Water | 53.25 | 43.08 | 47.27 | 225.4 | 219.64 | 210.82 | 2008.76 |
| Dimethyl ether | 12.12 | 17.86 | 15.86 | 0.0 | 0.0 | 0.0 | 18.05 |

tr = trace (less than 0.005 detected)

TABLE 2B: CONVERSIONS AND SELECTIVITIES

| Temp | WHSV | HOS | Feed% | | | Conversion Carbon molar % | | Selectivities based on carbon molar converted | | | | | |
|---|---|---|---|---|---|---|---|---|---|---|---|---|---|
| °C | $h^{-1}$ | | MeOH | 1-Butene | Water | MeOH | $nC_4=$ | $C_{1/2}$ | $C_3$ | $i-C_4=$ | $C_5=$ branched | $C_5=$ linear | $C_6+$ |
| 300 | 0.8 | 1 | 82 | 18 | 0 | 55.4 | 83.7 | 0.5 | 2.0 | 5.9 | 34.0 | 10.1 | 40.6 |
| 300 | 0.8 | 4 | 82 | 18 | 0 | 39.6 | 83.2 | 0.3 | 1.7 | 4.8 | 31.5 | 10.2 | 48.4 |
| 300 | 0.8 | 6 | 82 | 18 | 0 | 43.8 | 71.9 | 0.4 | 2.2 | 7.3 | 42.9 | 12.5 | 40.7 |
| 300 | 0.7 | 1 | 29.0 | 6.4 | 64.6 | 59.5 | -18.4 | 1.8 | 6.5 | 22.2 | 34.1 | 5.1 | 4.8 |
| 300 | 0.7 | 4 | 29.0 | 6.4 | 64.6 | 58.6 | -19.7 | 1.7 | 5.5 | 18.9 | 47.0 | 6.9 | 7.4 |
| 300 | 0.7 | 6 | 29.0 | 6.4 | 64.6 | 56.7 | -34.6 | 1.6 | 8.3 | 29.4 | 48.6 | 7.4 | 9.9 |
| 414 | 20.9 | 4 | 11.3 | 22.6 | 66.1 | 32.3 | 41.6 | 0.6 | 6.4 | 32.0 | 21.3 | 8.8 | 26.0 |

- sign means n-butenes formed to a level exceeding the quantity fed

EP 0 489 497 A1

**Claims**

1. A process for the production of olefins which comprises passing an oxygenate- containing feedstock over a zeo type catalyst at a temperature greater than 200°C, characterised in that the feedstock comprising an olefin and, as oxygenate, methanol, formaldehyde and/or dimethylether, the zeo type catalyst is of TON-type structure, and the feedstock includes added water.

2. A process according to Claim 1, in which the oxygenate is methanol.

3. A process according to either Claim 1 or Claim 2, in which the olefin is a $C_3$ and/or $C_4$ olefin.

4. A process according to any one of Claims 1 to 3, in which the TON-type catalyst is an aluminosilicate.

5. A process according to any one of Claims 1 to 4, in which the TON-type catalyst is used in the hydrogen form.

6. A process according to any one of Claims 1 to 5, in which the water is from 5 to 70% of total molar content of the feedstock.

7. A process according to any one of Claims 1 to 6, in which the reaction temperature is in the range of from 250 to 600°C.

8. A process according to any one of Claims 1 to 7, in which the reaction is carried out at a pressure of from 2 to 10 bar absolute

9. A process according to any one of Claims 1 to 8, in which the reaction is carried out at a WHSV of from 0.1 to 1000 per hour.

**European Patent Office**

# EUROPEAN SEARCH REPORT

Application Number

EP 91 31 0185

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | FR-A-2 577 549 (INSTITUT FRANCAIS DU PETROLE)  * claims * | 1-2,4, 7-8 | C07C11/02 C07C1/20 |
| X | EP-A-0 088 965 (BASF)  * claims * | 1-2,4, 7-8 | |
| A,D | US-A-4 543 435 (GOULD ET AL) | | |
| A,D | US-A-4 579 999 (GOULD ET AL) | | |
| A,D | EP-A-0 065 400 (IMPERIAL CHEMICAL INDUSTRIES) | | |

TECHNICAL FIELDS SEARCHED (Int. Cl.5 )

C07C

The present search report has been drawn up for all claims

| Place of search | Date of completion of the search | Examiner |
|---|---|---|
| THE HAGUE | 03 FEBRUARY 1992 | J. VAN GEYT |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)